# EUROPEAN PATENT SPECIFICATION

(11) **EP 0 415 755 B1**
(45) Date of publication and mention of the grant of the patent: **20.12.1995**
(21) Application number: 90309492.8
(22) Date of filing: 30.08.1990
(51) Int. Cl.: C12Q 1/68

(54) **Process for controlling contamination of oligonucleotide-dependent nucleic acid amplification reactions**
Verfahren zur Überprüfung der Kontamination von oligonukleotidabhängigen Nukleinsäure-Amplifikationsreaktionen
Procédé pour contrôler la contamination des réactions d'amplification des acides nucléiques dépendant des oligonucléotides

(30) Priority: 01.09.1989 US 401840
(43) Date of publication of application: 06.03.1991
(73) Proprietor: LIFE TECHNOLOGIES INC., Gaithersburg, MD 20877 (US)
(72) Inventor: Berninger, Mark, Gaithersburg, Maryland 20877 (US)
(74) Representative: Sheard, Andrew Gregory

(56) References cited:
- EP-A- 0 401 307
- EP-A- 0 407 291
- WO-A-89/11548
- NATURE, vol. 339, 18th May 1989, pages 237-238; S. KWOK et al.: "Avoiding false positives with PCR"
- GENE, vol. 93, 1st September 1990, pages 125-128; M.C. LONGO et al.: "Use of uracil DNA glycosylase to control carry-over contamination in polymerase chain reactions"

## Description

The present invention relates to an improvement in processes which amplify nucleic acid sequences. In particular, the present invention discloses a method for preventing cross-contamination of nucleic acid samples by eliminating the products of an execution of an oligonucleotide-dependent nucleic acid amplification process that contaminate subsequent executions of the amplification process. Such an improvement ensures that the results of an amplification process do not reflect the presence of cross-contaminating nucleic acid template.

The polymerase chain reaction (PCR) procedure amplifies specific nucleic acid sequences through a series of manipulations including denaturation, annealing of oligonucleotide primers, and extension of the primers with DNA polymerase (Mullis, K.B. et al., US 4,683,202, US 4,683,195; Mullis, K.B., EP 201,184; Erlich, H., EP 50,424, EP 84,796, EP 258,017, EP 237,362; Erlich, H., US 4,582,788; Saiki, R. et al., US 4,683,194; Mullis, K.B. et al. Cold Spring Harbor Symp. Quant. Biol. 51:263 (1986); Saiki, R. et al. Science 230:1350 (1985); Saiki, R. et al. Science 231:487 (1988); Loh, E.Y. et al. Science 243:217 (1988)). These steps can be repeated many times, potentially resulting in large amplifications of the number of copies of the original specific sequence. It has been shown that even single copy DNA sequences can be amplified to produce hundreds of nanograms of product (Li, H. et al. Nature 335:414 (1988)).

Other known nucleic acid amplification procedures include transcription-based amplification systems (Kwoh, D. et al. Proc. Natl. Acad. Sci. USA 86:1173 (1989); Gingeras, T.R. et al., WO 88/10315). Schemes based on ligation of two (or more) oligonucleotides in the presence of nucleic acid having the sequence of the resulting "di-oligonucleotide", thereby amplifying the di-oligonucleotide, are also known (Wu, D.Y. and Wallace, R.B. Genomics 4:560 (1989)).

A consequence of amplification processes such as PCR is that the amplification products themselves can be substrates for subsequent PCR procedures. Furthermore, because the quantities of the amplification products can be large, and because the sensitivity of PCR is so great, the dispersal of even an extremely small fraction of a reaction such as a PCR reaction into the laboratory area potentially can lead to contamination of later attempts to amplify other samples, thereby resulting in false positives. Extreme care must be taken to avoid cross contamination (Kwok, S. and Higuchi, R. Nature 339:237 (1989)); this is very inconvenient and adds significantly to the cost of doing amplifications such as PCR.

EP-A-407291, which forms part of the state of the art by virtue of Article 54(3) EPC, discloses a method for amplification of DNA using primers having a phosphosylated 5' end. The products of the amplification are subsequently treated with a lambda exonuclease.

Thus a need exists for a routine, economical method of oligonucleotide-dependent nucleic acid amplification wherein such amplification may be performed without concern as to possible cross-contamination from previous amplifications.

The present invention involves a process for incorporating a desoxyuridine into DNA or RNA during oligonucleotide-dependent amplification procedures.

The present invention represents an improvement upon in vitro oligonucleotide-dependent, nucleic acid amplification procedures in general. In the methods of the invention, amplification products are made distinguishable from the nucleic acid substrate used to initiate the amplification in a manner which imparts distinct properties to the amplification products. Accordingly, prior to the start of a new amplification reaction, these distinct properties may be exploited so as to render former amplification products inactive as templates in subsequent amplification reactions.

Therefore, this invention is directed to:
a process for oligonucleotide-dependent amplification of one or more nucleic acid sequences in a sample, comprising the steps of:
(a) amplifying nucleic acid of a first sample, wherein such amplifying is dependent on one or more specific oligonucleotides, and wherein at least one of such specific oligonucleotides comprises deoxyuridine, and
(b) treating the nucleic acid of a second sample with uracil DNA glycosylase which renders nucleic acid containing such deoxyuridine substantially unamplifiable in an amplification dependent on such specific oligonucleotides and which does not substantially affect amplification of nucleic acid that does not contain said deoxyuridine; whereby amplified nucleic acid sequences derived from such first sample which contaminate such second sample are not further substantially amplified during amplification dependent on such specific oligonucleotides of the nucleic acid sequences of such second sample.

This invention is further directed to the above process wherein the amplifying is a process for amplifying at least one specific nucleic acid sequence contained in a nucleic acid or a mixture of nucleic acids wherein each nucleic acid consists of two separate complementary strands, of equal or unequal length, which process further comprises:
(e) treating the strands with two oligonucleotide primers, for each different specific sequence being amplified, under conditions such that for each different sequence being amplified an extension product of each primer is synthesized which is complementary to each nucleic acid strand, wherein such primers are selected so as to be sufficiently complementary to different strands of each specific sequence to hybridize therewith such that the extension product synthesized from one primer, when it is separated from its complement, can serve as a template for synthesis of the extension product of the other primer;
(f) separating the primer extension products from the templates on which they were synthesized to produce single-stranded molecules; and
(g) treating the single-stranded molecules generated from step (f) with the primers of step (e) under conditions that a primer extension product is synthesized using each of the single strands produced in step (f) as a template.

In addition this invention is directed to a process for amplifying one or more nucleic acid sequences in a sample, comprising the steps of:
(A) with a first sample containing a nucleic acid sequence or a mixture of nucleic acid sequences, wherein each nucleic acid sequence has two separate complementary strands of equal or unequal length:
   (a) treating such strands with two oligonucleotide primers, for each different specific sequence being amplified, wherein at least one primer comprises deoxyuridine, under conditions such that for each different sequence being amplified an extension product of each primer is synthesized which is complementary to each nucleic acid strand, wherein such primers are selected so as to be sufficiently complementary to different strands of each specific sequence to hybridize therewith such that the extension product synthesized from one primer, when it is separated from its complement, can serve as a template for synthesis of the extension product of the other primer;
   (b) separating the primer extension products from the templates on which they were synthesized to produce single-stranded molecules;
   (c) treating such single-stranded molecules generated from step (b) with the primers of step (a) under conditions such that a primer extension product is synthesized using each of the single strands produced in step (b) as a template;
   (d) repeating steps (a) through (c) at least once, thereby amplifying the specific nucleic acid sequence contained by such first sample; and
(B) with a second sample containing a nucleic acid sequence or a mixture of nucleic acid sequences, wherein each nucleic acid sequence has two separate complementary strands of equal or unequal length and wherein amplified nucleic acid sequences of step (A) may be present in such second sample:
   (e) treating the nucleic acid of a second sample with uracil DNA glycosylase which renders nucleic acid containing such deoxyuridine substantially unamplifiable in an amplification dependent on such specific primers and which does not substantially affect amplification of nucleic acid that does not contain the deoxyuridine;
      whereby any primer extension products of such first sample, amplified in step (A) and present in such second sample, are not further substantially amplified in step (B).
      The above process may also further comprise during steps (B) the steps of:
   (f) repeating steps (a) through (c) at least once, thereby amplifying any of the specific nucleic acid sequence contained by said second sample; and/or
   (g) terminating treatment of step (e).

Lastly, this invention is directed to a method for rendering amplified nucleic acid unamplifiable comprising the steps of:
(a) treating a nucleic acid strand with a primer, wherein the primer comprises deoxyuridine, under conditions such that an extension product is synthesized which is complementary to the nucleic acid strand, and
(b) treating the extension product of step (a) with uracil DNA glycosylase which removes the base of the deoxyuridine, thereby forming an apyrimidinic deoxyribose in a sugar-phosphate backbone in the extension product.

In the description that follows, a number of terms used in molecular biology and nucleic acid amplification technology are extensively utilized. In order to provide a clearer and consistent understanding of the specification and claims, including the scope to be given such terms, the following definitions are provided.

"Amplification", as used herein, refers to any in vitro process for increasing the number of copies of a nucleotide sequence or sequences. Nucleic acid amplification results in the incorporation of nucleotides into DNA or RNA. As used herein, one amplification reaction may consist of many rounds of DNA replication. For example, one PCR reaction may consist of 30-100 "cycles" of denaturation and replication.

"Nucleotide" as used herein, is a term of art that refers to a base-sugar-phosphate combination. Nucleotides are the monomeric units of nucleic acid polymers, i.e. of DNA and RNA. The term includes ribonucleoside triphosphates, such as rATP, rCTP, rGTP, or rUTP, and deoxyribonucleotide triphosphates, such as dATP, dCTP, dGTP, or dTTP. A "nucleoside" is a base-sugar combination, i.e. a nucleotide lacking phosphate.

"Exo-sample nucleotide", as used herein, refers to a nucleotide which is generally not found in the sequence to be amplified. For most DNA samples, deoxyuridine is an example of an exo-sample nucleotide. Although the triphosphate form of deoxyuridine, dUTP, is present in living organisms as a metabolic intermediate, it is rarely incorporated into DNA. When dUTP is incorporated into DNA, the resulting deoxyuridine is promptly removed in vivo by normal processes, e.g. processes involving the enzyme uracil DNA glycosylase (UDG). Thus, deoxyuridine occurs rarely or never in natural DNA. It is recognized that some organisms may naturally incorporate deoxyuridine into DNA. For nucleic acid samples of those organisms, deoxyuridine would not be considered an exo-sample nucleotide. The presence of deoxyuridine, or any other exo-sample nucleotide, may be determined readily using methods well known to the art.

"Uracil DNA glycosylase" (UDG), a term of art, refers to an enzyme which cleaves the glycosidic bond between the base uracil and the sugar deoxyribose, only when the monomeric nucleotide dUTP is incorporated into a DNA molecule, resulting in incorporation of a deoxyuridine moiety (Duncan, B. in The Enzymes 14:565 (1981), ed.: Boyer P). The enzyme does not act upon free dUTP, free deoxyuridine, or RNA (Duncan, supra).

"Incorporating" as used herein, means becoming part of a nucleic acid polymer.

"Terminating" as used herein, means causing a treatment to stop. The term includes means for both permanent and conditional stoppages. For example, if the treatment is enzymatic, a permanent stoppage would be heat denaturation; a conditional stoppage would be, for example, use of a temperature outside the enzyme's active range. Both types of termination are intended to fall within the scope of this term.

"Oligonucleotide" as used herein refers collectively and interchangeably to two terms of art, "oligonucleotide" and "polynucleotide". Note that although oligonucleotide and polynucleotide are distinct terms of art, there is no exact dividing line between them and they are used interchangeably herein.

"Oligonucleotide-dependent amplification" as used herein refers to amplification using an oligonucleotide or polynucleotide to amplify a nucleic acid sequence. An oligonucleotide-dependent amplification is any amplification that requires the presence of one or more oligonucleotides or polynucleotides that are two or more mononucleotide subunits in length and that end up as part of the newly-formed, amplified nucleic acid molecule.

"Primer" as used herein refers to a single-stranded oligonucleotide or a single-stranded polynucleotide that is extended by covalent addition of nucleotide monomers during amplification. Nucleic acid amplification often is based on nucleic acid synthesis by a nucleic acid polymerase. Many such polymerases require the presence of a primer that can be extended to initiate such nucleic acid synthesis.

The methods of the invention allow amplification products to be distinguished from the original nucleic acid substrate in a manner which does not interfere with detection analysis of such amplification products but which does make such amplification products uniquely susceptible to certain treatments which destroy their ability to serve as templates in subsequent amplification reactions. Because of this susceptibility, pretreating samples to be amplified according to the methods of the invention allows the elimination of any contaminating products of previous amplifications from the sample.

A similar approach to that presented herein is used by Hartley, J.L., U.S. Ser. No. 07/360,120, filed 1 June 1989, which is equivalent to US-A-5 035 996, wherein an exo-sample nucleotide is incorporated into amplification products during the amplification procedure itself so as to distinguish such amplified sequences from the original template.

The present invention is distinct from that of Hartley, as the deoxyuridine used to distinguish such amplification products herein is provided as part of an oligomer or polymer before amplification, while that of Hartley is provided as a nucleoside triphosphate which becomes incorporated into polymeric nucleic acid during amplification.

The methods of the invention eliminate contamination of starting materials with the end products of previous amplification processes as a major problem of nucleic acid amplification techniques.

The methods of the invention are applicable to any in vitro procedures which utilize enzymes to amplify specific nucleic acid sequences and especially to PCR.

In a preferred embodiment, the nucleic acid products produced by an amplification which uses primers containing deoxyuridines are chemically different from starting templates which were not produced by an amplification containing exo-sample nucleotides. This chemical difference permits one to render nucleic acid products, which were produced by an amplification which used primers containing deoxyuridines incapable of further exponential amplification. Incorporation of a deoxyuridine into a primer allows the DNA or RNA produced during such amplification processes to be differentiated from the original nucleic acids present in the sample prior to amplification. If desired, the amplification reaction itself may, in addition, further provide deoxyuridines for incorporation into the replicating nucleic acid, for example, as taught by Hartley, supra.

Typically, primers are used wherein the ribonucleotide, UTP, or deoxyribonucleotide, dTTP, in the oligonucleotide is replaced with one or more deoxyuridines. Embodiments utilizing primers with high proportions of deoxyuridine are preferred over those with fewer deoxyuridine-containing oligonucleotides. Especially, primers with a high fraction of deoxyuridine located at the 3'OH end of the primer are preferred. In another preferred embodiment, deoxyuridine is the 3' nucleotide.

In one embodiment, treatment with deoxyuridine primers to render amplified nucleic acid un-amplifiable is performed before beginning an amplification reaction.

In another embodiment, treatment with deoxyuridine primers to render amplified nucleic acid unamplifiable is performed after terminating a first amplification reaction and after removing a sample of the amplified products for further analysis, but before subjecting the original, starting nucleic acid in the sample to a new, second amplification reaction. For example, after amplification and after removing amplified samples for analysis, amplified product that may be remaining is subjected to a treatment which renders amplified nucleic acid containing the deoxyuridine substantially unamplifiable.

Such treatment to render the amplified nucleic acid containing the deoxyuridine unamplifiable is a uracil DNA glycosylase treatment. Such treatment may be done as a separate step or, preferably, may be done in the presence of a second sample containing nucleic acid sequences to be amplified. Accordingly, amplified nucleic acid sequences produced by amplification of a first sample in the presence of deoxyuridine which contaminate a second sample are not further substantially amplified during the second oligonucleotide-dependent amplification of nucleic acid sequences even if the same oligonucleotides(s) are used as primers.

The deoxyribonucleotide deoxyuridine is used as an exo-sample nucleotide which may be conveniently incorporated into primers used in an oligonucleotide-dependent DNA amplification procedure, exemplified herein by PCR, thereby resulting in deoxyuridine-containing, DNA amplification products. The DNA products of such a reaction will normally contain as many uracil bases as would be present thymines in a non-exo-nucleotide-containing primer.

Discrimination between a nucleic acid which does not contain the exo-nucleotide deoxyuridine and a deoxyuridine-containing product of an amplification reaction is obtained with the enzyme UDG. Treatment of DNA containing uracil bases with UDG results in cleavage of the glycosidic bond between the deoxyribose of the DNA sugar-phosphate backbone and the uracil base. The loss of the uracil creates an apyrimidinic site in the DNA, which blocks DNA polymerase from using the DNA strand as a template for the synthesis of a complementary DNA strand (Schaaper, R. et al. Proc. Natl. Acad. Sci. USA 80:487 (1983)).

By providing primers containing deoxyuridine, such deoxyuridines are localized at the 5' ends of each strand of DNA template which is amplified. When deoxyuridine-containing primers have been used and the sample treated with UDG, substantial numbers of apyrimidinic sites in the 5'-end of each DNA target template molecule are found. Such apyrimidinic sites interfere with synthesis of sequences at the 3'-end of newly made strands. These 3'-end sequences are the targets to which original deoxyuridine-containing primers bind. Thus these primers do have target sequences with which they can bind an nucleic acid derived from molecules primed by deoxyuridine-containing primers.

As exemplified herein, in a preferred embodiment, the basic amplification protocol PCR is modified in three ways: (1) amplification is first performed using oligonucleotide primers containing deoxyuracil substituted for deoxythymidine and samples of the amplified products are removed for analysis; (2) UDG is then added to the PCR reaction mixture; and (3) an incubation period is added to allow UDG to act on uracil-containing sequences in contaminating products of prior PCR reactions.

Before continuing with the amplification procedure, the UDG must be inactivated. UDG may be either permanently inactivated by high temperature in the first PCR cycle or by the high temperatures used with Taq DNA polymerase in the currently preferred PCR protocol. This inactivation prevents UDG from destroying newly-synthesized PCR products. Nucleic acid amplification protocols that do not inherently eliminate UDG activity usually will require an extra UDG-inactivation step.

Embodiments having a greater proportion of oligonucleotides containing deoxyuridines are preferred. However, even in standard PCR embodiments that depend on two oligonucleotide primers, the present invention is capable of rendering PCR contaminants unamplifiable as long as at least one primer contains deoxyuridine.

Not all single deoxyuridine-containing primers may reduce amplification of PCR products to sufficiently low levels for a given situation; those of ordinary skill in the art can empirically find which primers are acceptable during routine optimization and testing of a PCR assay, without undue experimentation.

Routine assay optimization, aimed at testing oligonucleotide suitability, can be done by (1) making a deoxyuridine oligonucleotide, (2) performing a first nucleic acid amplification of a target sequence using that primer, (3) seeding various amounts of the resulting first product in a new, second amplification that does not contain target sequences, (4) treating the second amplification with uracil DNA glycosylase to render exo-sample nucleotide-containing nucleic acid unamplifiable, (5) performing the second amplification, and (6) assaying the resultant second product for presence of contaminating sequence from the first hybridization. All of these steps are routinely done as experimental controls and as part of the normal validation of a method. The only additional work required of one practicing the present invention involves routine synthesis of any additional oligonucleotides that may be required. Generally, if a oligonucleotide is observed to be not suitable, a substitute can be easily found and similarly tested.

As will be understood by those of ordinary skill in the art, the methods of the present invention prevent exponential amplification but may not prevent linear amplification. Linear amplification does usually not represent a substantial problem in amplification procedures. For example, a single amplification product molecule which contaminates a sample run through 20 cycles of PCR, amplifying by a factor of two on each cycle, will result in only about 20 molecules if amplified linearly but could result in up to about a million molecules if amplified exponentially at maximal theoretical efficiency. Thus, linear amplification is generally inconsequential.

The present invention can be adapted to be used with amplifications that do not use a nucleic acid polymerase. For instance, contamination of samples with previously made products can be controlled by amplification schemes that polymerize oligonucleotides with ligase (e.g. Wu and Wallace, supra).

Having now generally described this invention, the same will be better understood by reference to certain examples which are included herein for purposes of illustration only and is not intended to be limiting unless otherwise specified.

### EXAMPLE 1

### General Experimental Conditions

Polymerase chain reactions (PCRs) were performed to amplify a region of the human papilloma virus type 16 (HPV 16) DNA (Durst, M. et al. Proc. Natl. Acad. Sci. USA 80:3812 (1983)). The sequences of the oligonucleotide primers used were either 5'GGUCGAUGUAUGUCUUGUUG3' and 5'GUCUACGUGUGUGCUUUGUAC3' (dU primers dU₁ and dU₂, respectively) or 5'GGTCGATGTATGTCTTGTTG3' and 5'GTCTACGTGTGTGCTTTGTAC3' (control dT primers dT₁ and dT₂, respectively). Note that the sequences of dT₁ and dU₂ were identical except for the replacement of U for T; dT₂ and dU₂ were similarly identical.

HPV 16 DNA was excised from a full length plasmid clone, pT7HPV16 (for the purposes of this invention, equivalent to the pUC8-based plasmids described by Seedoff, K. et al. Virol. 145:181 (1985)) with the restriction enzyme BamH I. The linear DNA (10 picograms) was added to PCR reactions containing 50 microliters of 25 mM Tris HCl pH 8.3, 5 mM MgCl₂, 50 mM NaCl, 0.01% gelatin, 0.2 mM each dATP, dGTP, dCTP, 0.2 mM either dUTP or dTTP, 1 micromolar of each primer, and 2.5 units of thermostable DNA polymerase from Thermus aquaticus (Cetus/Perkin-Elmer). Primers and triphosphates were paired as follows: dU primers with dTTP (pUxT reactions ("primed with U, extended with T")) and control dT primers with dUTP (pTxU reactions). The reactions were amplified in a thermal cylcer (Cetus/Perkin-Elmer) using the following temperature profile: 5 minutes at 94°C, then 30 cycles of 1 minute at 94°C (denaturation), two minutes at 55°C (annealing), and 3 minutes at 72°C (primer extension). After completion of the temperature cycles, a final extension of 10 minutes at 72°C was done. Amplification of the 284 base pair HPV 16 DNA fragment was confirmed by agarose/ethidium bromide gel electrophoresis (Maniatis, T. et al. (1982) Molecular Cloning, Cold Spring Harbor Laboratory) of the PCR reaction products (5 microliters of each reaction per lane). All reactions showed substantial amplification. Negative control reactions to which no HPV 16 DNA was added did not produce any DNA products. The concentration of the PCR amplification products was estimated from the agarose gel.

### EXAMPLE 2

### Amplification Using Two Deoxyuridine-Containing Primers

New pUxT PCR reactions were prepared that contained dTTP but lacked dU primers These were contaminated with ten femtograms of pUxT amplification products from the previous reactions, and incubated for 15 minutes at 37 C in the presence or absence of UDG (J. Van de Sande, University of Calgary; also available from Duncan Laboratories, 10 E. Central Ave., Paoli, PA 19301 USA). These reactions were then incubated at 94 C to inactivate the UDG, cooled to 15 C, and dU primers were added.

New pTxU PCR reactions were prepared with dT primers and dUTP and were contaminated with ten femtograms of pTxU amplification products. These reactions were incubated at 37 C for 15 minutes in the presence or absence of UDG to act as controls for UDG activity and PCR amplification, respectively.

The new PCR reactions were then subjected to the same PCR temperature cycling described above. Agarose/ethidium bromide gel electrophoresis showed that in the absence of UDG treatment both pUxT and pTxU reaction products could be reamplified by PCR to give results identical in appearance to those obtained upon amplification of HPV 16 DNA. In contrast, UDG treatment abolished reamplification of both pTxU reaction products (the positive control) and pUxT reaction products, which contained dU primers. In another experiment it was shown that, in the absence of deoxyuridine in either the primers or extension products, UDG did not affect the PCR amplification of natural HPV 16 DNA.

Without UDG treatment, the deoxyuridine-containing PCR products could be re-amplified to give a DNA product indistinguishable in size, as evidenced by agarose gel electrophoresis, from the products which did not contain deoxyuridine and which were obtained by amplifying the normal HPV 16 DNA. The reaction in which the DNA was made with deoxyuridine-containing primers and which was incubated with UDG prior to PCR did not give any visible products on the agarose gel. In another experiment it was shown that UDG did not affect the amplification of natural DNA.

### EXAMPLE 3

### Amplification Using a Single Deoxyuridine-Containing Primer

A further experiment demonstrated that only one of two PCR primers need contain an exo-sample nucleotide. PCR amplifications were done essentially as described above using the following pairs of primers: (1) dT₁ with dT₂, (2) dT₁ with dU₂, (3) dU₁ with dT₂, and (4) dU₁ with dU₂. All combinations were treated both with and without UDG before the second round of PCR. Amplification (1) tested for success of PCR amplification while amplification (4) tested for activity of UDG and the ability to eliminate contamination. Amplification (3) was observed to not be substantially reamplified after UDG treatment in a second round of PCR, thereby demonstrating that only one of a pair of PCR primers need contain an exo-sample nucleotide in order to reduce or eliminate the effects of sample contamination. Amplification (2) was observed to be reamplified, though at a substantially lower level than amplification (1).

### Summary

In summary, PCR amplification products that contained deoxyuridine in a primer or primers were successfully amplified if no UDG was present; amplification could be prevented by prior incubation with UDG. In other words, UDG could substantially abolished amplification of PCR products made with deoxyuridine-containing primers but had no substantial effect on the amplification of DNA made with deoxythymidine-containing primers.

## Claims

1. A process for oligonucleotide-dependent amplification of one or more nucleic acid sequences in a sample, comprising the steps of:
(a) amplifying nucleic acid of a first sample, wherein said amplifying is dependent on one or more specific oligonucleotides, and wherein at least one of said specific oligonucleotides comprises deoxyuridine;
(b) treating the nucleic acid of a second sample with uracil DNA glycosylase which renders nucleic acid containing said deoxyuridine substantially unamplifiable in an amplification dependent on said specific oligonucleotides and which does not prevent amplification of nucleic acid that does not contain said deoxyuridine;
whereby amplified nucleic acid sequences derived from said first sample which contaminate said second sample are not further substantially amplified during amplification dependent on said specific oligonucleotides of the nucleic acid sequences of said second sample.

2. A process as claimed in claim 1, further comprising the step of:
(c) amplifying nucleic acid sequences of said second sample.

3. A process as claimed in claim 1 or claim 2 further comprising the step of:
(d) terminating said treatment of step (b).

4. A process as claimed in claims 1 to 3 wherein the amplifying is a process for amplifying at least one specific nucleic acid sequence contained in a nucleic acid or a mixture of nucleic acids wherein each nucleic acid consists of two separate complementary strands, of equal or unequal length, which process further comprises:
(e) treating the strands with two oligonucleotide primers, for each different specific sequence being amplified, under conditions such that for each different sequence being amplified an extension product of each primer is synthesized which is complementary to each nucleic acid strand, wherein said primers are selected so as to be sufficiently complementary to different strands of each specific sequence to hybridize therewith such that the extension product synthesized from one primer, when it is separated from its complement, can serve as a template for synthesis of the extension product of the other primer;
(f) separating the primer extension products from the templates on which they were synthesized to produce single-stranded molecules; and
(g) treating the single-stranded molecules generated from step (f) with the primers of step (e) under conditions that a primer extension product is synthesized using each of the single strands produced in step (f) as a template.

5. A process for amplifying one or more nucleic acid sequences in a sample, comprising the steps of:
(A) with a first sample containing a nucleic acid sequence or a mixture of nucleic acid sequences, wherein each nucleic acid sequence has two separate complementary strands of equal or unequal length:
(a) treating said strands with two oligonucleotide primers, for each different specific sequence being amplified, wherein at least one primer comprises deoxyuridine, under conditions such that for each different sequence being amplified an extension product of each primer is synthesized which is complementary to each nucleic acid strand, wherein said primers are selected so as to be sufficiently complementary to different strands of each specific sequence to hybridize therewith such that the extension product synthesized from one primer, when it is separated from its complement, can serve as a template for synthesis of the extension product of the other primer;
(b) separating the primer extension products from the templates on which they were synthesized to produce single-stranded molecules;
(c) treating said single-stranded molecules generated from step (b) with the primers of step (a) under conditions such that a primer extension product is synthesized using each of the single strands produced in step (b) as a template;
(d) repeating steps (a) through (c) at least once, thereby amplifying the specific nucleic acid sequence contained by said first sample; and
(B) with a second sample containing a nucleic acid sequence or a mixture of nucleic acid sequences, wherein each nucleic acid sequence has two separate complementary strands of equal or unequal length and wherein amplified nucleic acid sequences of step (A) may be present in said second sample:
(e) treating the nucleic acid of a second sample with uracil DNA glycosylase which renders nucleic acid containing said deoxyuridine substantially unamplifiable in an amplification dependent on said specific primers and which does not substantially affect amplification of nucleic acid that does not contain the deoxyuridine;
whereby any primer extension products of said first sample, amplified in step (A) and present in said second sample, are not further substantially amplified in step (B).

6. A process as claimed in claim 5, further comprising during steps (B) the step of:
(f) repeating steps (a) through (c) at least once, thereby amplifying any of the specific nucleic acid sequence contained by said second sample;

7. A process as claimed in claim 5 or claim 6, further comprising during steps (B) the step of:
(g) terminating treatment of step (e).

8. A process as claimed in claim 4 or claim 7, wherein the termination of the treatment is accomplished by heating.

9. A process as claimed in claims 1-8, wherein at least two of said oligonucleotide primers comprise deoxyuridine.

10. A method for rendering amplified nucleic acid unamplifiable, comprising the steps of:
(a) treating a nucleic acid strand with a primer, wherein the primer comprises deoxyuridine, under conditions such that an extension product is synthesized which is complementary to the nucleic acid strand, and
(b) treating the extension product of step (a) with uracil DNA glycosylase which removes the base of the deoxyuridine, thereby forming an apyrimidinic deoxyribose in a sugar-phosphate backbone in the extension product.

## Patentansprüche

1. Verfahren zur oligonucleotidabhängigen Amplifikation einer oder mehrerer Nucleinsäuresequenzen in einer Probe, das folgende Schritte umfaßt:
(a) Amplifizieren der Nucleinsäure einer ersten Probe, wobei das Amplifizieren von einem oder mehreren spezifischen Oligonucleotiden abhängig ist und wobei zumindest eines der spezifischen Oligonucleotide Desoxyuridin umfaßt;
(b) Behandeln der Nucleinsäure einer zweiten Probe mit Uracil-DNA-Glycosylase, wodurch Desoxyuridin-haltige Nucleinsäure in einer von den spezifischen Oligonucleotiden abhängigen Amplifikation, im wesentlichen nicht amplifizierbar gemacht wird und die Amplifikation der Nucleinsäure, die kein Desoxyuridin enthält, nicht verhindert wird;
wobei amplifizierte, von der ersten Probe abgeleitete Nucleinsäuresequenzen, welche die zweite Probe kontaminieren, während der von den spezifischen Oligonucleotiden der Nucleinsäuresequenzen der zweiten Probe abhängigen Amplifikation, im wesentlichen nicht weiter amplifiziert werden.

2. Verfahren nach Anspruch 1, das ferner den folgenden Schritt umfaßt:
(c) Amplifizieren von Nucleinsäuresequenzen der zweiten Probe.

3. Verfahren nach Anspruch 1 oder Anspruch 2, das ferner den folgenden Schritt umfaßt:
(d) Beenden der Behandlung von Schritt (b).

4. Verfahren nach den Ansprüchen 1 bis 3, wobei das Amplifizieren ein Verfahren zur Amplifikation von zumindest einer spezifischen, in einer Nucleinsäure oder einer Mischung von Nucleinsäuren enthaltenen Nucleinsäuresequenz ist, wobei jede Nucleinsäure aus zwei getrennten komplementären Strängen gleicher oder ungleicher Länge besteht, wobei das Verfahren ferner umfaßt:
(e) Behandeln der Stränge mit zwei Oligonucleotid-Primern für jede unterschiedliche, spezifische Sequenz, die amplifiziert wird, unter solchen Bedingungen, daß für jede unterschiedliche Sequenz, die amplifiziert wird, ein Extensionsprodukt jedes Primers synthetisiert wird, das zu jedem Nucleinsäurestrang komplementär ist, wobei die Primer derart gewählt werden, daß sie zu unterschiedlichen Strängen jeder spezifischen Sequenz ausreichend komplementär sind, um mit diesen zu hybridisieren, so daß das Extensionsprodukt, das von einem Primer synthetisiert wird, wenn es von seinem Komplement getrennt wird, als Matrize zur Synthese des Extensionsproduktes des anderen Primers dienen kann;
(f) Abtrennen der Primerextensionsprodukte von den Matrizen, auf welchen sie synthetisiert wurden, um einzelsträngige Moleküle herzustellen; und
(g) Behandeln der einzelsträngigen Moleküle, die in Schritt (f) erhalten wurden, mit den Primern von Schritt (e) unter solchen Bedingungen, daß ein Primerextensionsprodukt unter Verwendung jedes der Einzelstränge, die in Schritt (f) hergestellt wurden, als Matrize synthetisiert wird.

5. Verfahren zur Amplifikation einer oder mehrerer Nucleinsäuresequenzen in einer Probe, das die folgenden Schritte umfaßt:
(A) bei einer ersten Probe, die eine Nucleinsäuresequenz oder eine Mischung von Nucleinsäuresequenzen enthält, wobei jede Nucleinsäure zwei getrennte komplementäre Strängen gleicher oder ungleicher Länge aufweist:
(a) Behandeln der Stränge mit zwei Oligonucleotidprimern für jede unterschiedliche spezifische Sequenz, die amplifiziert wird, wobei zumindest ein Primer Desoxyuridin umfaßt, unter solchen Bedingungen, daß für jede unterschiedliche Sequenz, die amplifiziert wird, ein Extensionsprodukt jedes Primers synthetisiert wird, das zu jedem Nucleinsäurestrang komplementär ist, wobei die Primer derart gewählt werden, daß sie zu unterschiedlichen Strängen jeder spezifischen Sequenz ausreichend komplementär sind, um mit diesen zu hybridisieren, so daß das Extensionsprodukt, das von einem Primer synthetisiert ist, wenn es von seinem Komplement getrennt wird, als Matrize zur Synthese des Extensionsproduktes des anderen Primers dienen kann;
(b) Abtrennen der Primerextensionsprodukte von den Matrizen, auf welchen sie synthetisiert wurden, um einzelsträngige Moleküle herzustellen;
(c) Behandeln der einzelsträngigen Moleküle, die in Schritt (b) erhalten wurden, mit den Primern von Schritt (a) unter solchen Bedingungen, daß ein Primerextensionsprodukt unter Verwendung jedes der Einzelstränge, die in Schritt (b) hergestellt wurden, als Matrize synthetisiert wird;
(d) zumindest einmaliges Wiederholen der Schritte (a) bis (c), wodurch die spezifische Nucleinsäuresequenz, die in der ersten Probe enthalten ist, amplifiziert wird; und
(B) bei einer zweiten Probe, die eine Nucleinsäuresequenz oder eine Mischung von Nucleinsäuresequenzen enthält, wobei jede Nucleinsäure zwei getrennte komplementäre Strängen gleicher oder ungleicher Länge aufweist und wobei amplifizierte Nucleinsäuresequenzen von Schritt (A) in der zweiten Probe vorhanden sein können:
(e) Behandeln der Nucleinsäure einer zweiten Probe mit Uracil-DNA-Glycosylase, wodurch Desoxyuridin-haltige Nucleinsäure in einer von den spezifischen Primern abhängigen Amplifikation, im wesentlichen nicht amplifizierbar wird und die Amplifikation der Nucleinsäure, die kein Desoxyuridin enthält, im wesentlichen nicht beeinträchtigt wird;
wobei alle Primerextensionsprodukte der ersten Probe, die in Schritt (A) amplifiziert wurden und in der zweiten Probe vorhanden sind, in Schritt (B) im wesentlichen nicht weiter amplifiziert werden.

6. Verfahren nach Anspruch 5, das ferner während der Schritte (B) den folgenden Schritt umfaßt:
(f) zumindest einmaliges Wiederholen der Schritte (a) bis (c), wodurch jede spezifische Nucleinsäuresequenz, die in der zweiten Probe enthalten ist, amplifiziert wird.

7. Verfahren nach Anspruch 5 oder Anspruch 6, das ferner während der Schritte (B) den folgenden Schritt umfaßt:
(g) Beenden der Behandlung von Schritt (e).

8. Verfahren nach Anspruch 4 oder Anspruch 7, wobei das Beenden der Behandlung durch Erwärmen erfolgt.

9. Verfahren nach den Ansprüchen 1 bis 8, wobei zumindest zwei der Oligonucleotidprimer Desoxyuridin umfassen.

10. Verfahren, um amplifizierte Nucleinsäure nicht amplifizierbar zu machen, das folgende Schritte umfaßt:
(a) Behandeln eines Nucleinsäurestranges mit einem Primer, wobei der Primer Desoxyuridin umfaßt, unter solchen Bedingungen, daß ein Extensionsprodukt synthetisiert wird, das zu dem Nucleinsäurestrang komplementär ist, und
(b) Behandeln des Extensionsproduktes von Schritt (a) mit Uracil-DNA-Glycosylase, welche die Base des Desoxyuridins entfernt, wodurch eine apyrimidinische Desoxyribose in einer Zucker-Phosphat-Hauptkette in dem Extensionsprodukt gebildet wird.

## Revendications

1. Procédé d'amplification dépendant d'oligonucléotides d'une ou de plusieurs séquences d'acide nucléique dans un échantillon, comprenant les étapes de :
(a) amplification de l'acide nucléique d'un premier échantillon, où l'amplification dépend d'un ou de plusieurs oligonucléotides spécifiques et où au moins l'un des oligonucléotides spécifiques comprend de la désoxyuridine;
(b) traitement de l'acide d'un deuxième échantillon avec de l'uracile-ADNglycosylase qui rend l'acide nucléique contenant la désoxyuridine, sensiblement non amplifiable dans une amplification dépendant des oligonucléotides spécifiques et qui ne prévient pas l'amplification d'acide nucléique qui ne contient pas de désoxyuridine;
où les séquences d'acide nucléique amplifiées provenant du premier échantillon, qui contaminent le deuxième échantillon ne sont plus sensiblement amplifiées pendant l'amplification dépendant des oligonucléotides spécifiques des séquences d'acide nucléique du deuxième échantillon.

2. Procédé suivant la revendication 1, comprenant, en outre, l'étape de :
(c) amplification des séquences d'acide nucléique du deuxième échantillon.

3. Procédé suivant la revendication 1 ou 2, comprenant, en outre, l'étape de :
(d) terminaison du traitement de l'étape b).

4. Procédé suivant les revendications 1 à 3, dans lequel l'amplification est un procédé d'amplification d'au moins une séquence spécifique d'acide nucléique, contenue dans un acide nucléique ou un mélange d'acides nucléiques où chaque acide nucléique consiste en deux brins complémentaires séparés, de longueur égale ou inégale, lequel procédé comprend, en outre :
(e) le traitement des brins au moyen de deux amorces oligonucléotidiques, pour chaque séquence spécifique différente à amplifier, dans des conditions telles que pour chaque séquence différente à amplifier, un produit d'extension de chaque amorce est synthétisé, qui est complémentaire de chaque brin d'acide nucléique, où les amorces sont choisies de manière à être suffisamment complémentaires des différents brins de chaque séquence spécifique pour s'y hybrider, de sorte que le produit d'extension synthétisé à partir d'une amorce, lorsqu'il est séparé de son complément, peut servir de matrice à la synthèse du produit d'extension de l'autre amorce;
(f) la séparation des produits d'extension d'amorce des matrices sur lesquelles ils ont été synthétisés pour produire des molécules simple brin, et
(g) le traitement des molécules simple brin générées à l'étape (f) avec les amorces de l'étape (e) dans des conditions telles qu'un produit d'extension d'amorce soit synthétisé au moyen de chacun des simples brins produits à l'étape (f) comme matrice.

5. Procédé d'amplification d'une ou de plusieurs séquences d'acide nucléique dans un échantillon, comprenant les étapes de :
(A) avec un premier échantillon contenant une séquence d'acide nucléique ou un mélange de séquences d'acide nucléique, où chaque séquence d'acide nucléique a deux brins complémentaires de longueur égale ou inégale :
(a) traitement des brins avec deux amorces oligonucléotidiques pour chaque séquence spécifique à amplifier, où au moins l'une des amorces comprend de la désoxyuridine, dans des conditions telles que pour chaque séquence différente à amplifier, un produit d'extension de chaque amorce est synthétisé, qui est complémentaire de chaque brin d'acide nucléique, où les amorces sont choisies de manière à être suffisamment complémentaires des différents brins de chaque séquence spécifique pour s'y hybrider, de sorte que le produit d'extension synthétisé à partir d'une amorce, lorsqu'il est séparé de son complément, puisse servir comme matrice à la synthèse du produit d'extension de l'autre amorce;
(b) séparation des produits d'extension d'amorce des matrices sur lesquelles ils ont été synthétisés pour produire des molécules simple brin;
(c) traitement des molécules simple brin générées à l'étape (b) avec les amorces de l'étape (a) dans des conditions telles qu'un produit d'extension d'amorce soit synthétisé au moyen de chacun des simples brins produits à l'étape (b) comme matrice;
(d) répétition des étapes (a) à (c) au moins une fois, pour ainsi amplifier la séquence spécifique d'acide nucléique contenue dans le premier échantillon, et
(B) avec un deuxième échantillon contenant une séquence d'acide nucléique ou un mélange de séquences d'acide nucléique, où chaque séquence d'acide nucléique a deux brins complémentaires de longueur égale ou inégale et où les séquences d'acide nucléique amplifiées à l'étape (A) peuvent être présentes dans le deuxième échantillon :
(e) traitement de l'acide nucléique d'un deuxième échantillon avec de l'uracile-ADNglycosylase qui rend l'acide nucléique contenant la désoxyuridine, sensiblement non amplifiable dans une amplification dépendant des amorces spécifiques et qui n'affecte sensiblement pas l'amplification d'acide nucléique qui ne contient pas de désoxyuridine;
où tout produit d'extension d'amorce du premier échantillon, amplifié à l'étape (A) et présent dans le deuxième échantillon, n'est plus sensiblement amplifié pendant l'étape (B).

6. Procédé suivant la revendication 5, qui comprend, en outre, pendant les étapes (B), l'étape de :
(f) répétition des étapes (a) à (c) au moins une fois, pour amplifier ainsi l'une quelconque des séquences spécifiques d'acide nucléique contenues dans le deuxième échantillon.

7. Procédé suivant la revendication 5 ou 6, qui comprend, en outre, pendant les étapes (B), l'étape de :
(g) terminaison du traitement de l'étape (e).

8. Procédé suivant la revendication 4 ou 7, dans lequel la terminaison du traitement est réalisée par chauffage.

9. Procédé suivant les revendications 1 à 8, dans lequel au moins deux des amorces oligonucléotidiques comprennent de la désoxyuridine.

10. Procédure pour rendre un acide nucléique amplifié non amplifiable, comprenant les étapes de :
(a) traitement d'un brin d'acide nucléique avec une amorce, où l'amorce comprend de la désoxyuridine, dans des conditions telles qu'un produit d'extension est synthétisé, qui est complémentaire du brin d'acide nucléique, et
(b) traitement du produit d'extension de l'étape (a) avec de l'uracile-ADNglycosylase qui élimine la base de la désoxyuridine, pour ainsi former un désoxyribose apyrimidinique dans un squelette sucre-phosphate dans le produit d'extension.
